# EUROPEAN PATENT SPECIFICATION

(11) **EP 1 660 676 B1**
(45) Date of publication and mention of the grant of the patent: **07.01.2009**
(21) Application number: 04768320.6
(22) Date of filing: 02.09.2004
(51) Int. Cl.: C12Q 1/68

(54) **DIAGNOSIS OF RISK OF BREAST CANCER**
DIAGNOSE DES BRUSTKREBSRISIKOS
DIAGNOSTIC DU RISQUE DE CANCER DU SEIN

(30) Priority: 03.09.2003 GB 0320648
(43) Date of publication of application: 31.05.2006
(73) Proprietor: Randox Laboratories Ltd., Crumlin, Co. Antrim BT29 4QY (GB)
(72) Inventor: CROCKARD, Martin Andrew Randox Laboratories Ltd., Co. Antrim BT29 4QY (GB); BAILIE, Janice Roberta Randox Laboratories Ltd., Co. Antrim BT29 4QY (GB); LAMONT, John Victor Randox Laboratories Ltd., Co. Antrim BT29 4QY (GB); FITZGERALD, Stephen Peter Randox Laboratories Ltd., Co. Antrim BT29 4QY (GB)
(74) Representative: Jappy, John William Graham
(86) International application number: PCT/GB2004/003773
(87) International publication number: WO 2005/024060

(56) References cited:
- WO-A-01/92581
- WO-A-02/092854
- WO-A-03/061386

## Description

### Field of the Invention

This invention relates to the detection of the presence of or the risk of breast cancer.

### Background of the Invention

There are over 1 million cases of breast cancer per year on a global basis, of which around 0.5 million are in the US, 40,000 are in the UK and nearly 2,000 in Ireland. It is the leading cause of cancer deaths among women. Although the overall incidence of the disease is increasing within the western world, wider screening and improved treatments have led to a gradual decline in the fatality rate of about 1% per year since 1991. Patients diagnosed with early breast cancer have greater than a 90% 5 year relative survival rate, as compared to 20% for patients diagnosed with distally metastasised breast cancer. Nonetheless, there is no definitive early-stage screening test for breast cancer, diagnosis currently being made on the results of mammography and fine needle biopsy. Mammography has its limitations, with over 80% of suspicious results being false positives and 10-15% of women with breast cancer providing false negative results. Often the tumour has reached a late stage in development before detection, reducing the chances of survival for the patient and increasing the cost of treatment and management for the healthcare system. More sensitive methods are required to detect small (<2 cm diameter) early stage *in-situ* carcinomas of the breast, to reduce patient mortality. In addition to early detection, there remain serious problems in classifying the disease as malignant or benign, in the staging of known cancers and in differentiating between tumour types. Finally, there is a need to monitor ongoing treatment effects and to identify patients becoming resistant to particular therapies. Such detection processes are further complicated, as the mammary gland is one of the few organs that undergoes striking morphological and functional changes during adult life, particularly during pregnancy, lactation and involution, potentially leading to changes in the molecular signature of the same mammary gland over time.

Diagnosis of disease is often made by the careful examination of the relative levels of a small number of biological markers. Despite recent advances, the contribution of the current biomarkers to patient care and clinical outcome is limited. This is due to the low diagnostic sensitivity and disease specificity of the existing markers. Some molecular biomarkers, however, are being used routinely in disease diagnosis, for example prostate specific antigen in prostate cancer screening, and new candidate markers are being discovered at an increasing rate (Pritzker, 2002). It is becoming accepted that the use of a panel of well-validated biomarkers would enhance the positive predictive value of a test and minimize false positives or false negatives (Srinivas et al., 2002). In addition, there is now growing interest in neural networks, which show the promise of combining weak but independent information from various biomarkers to produce a prognostic/predictive index that is more informative than each biomarker alone (Yousef et al., 2002).

WO-A-01/92581 discloses polynucleotide sequences that are useful in the therapy and diagnosis of cancer, particularly ovarian cancer. WO-A-03/061386 discloses methods for inhibiting the growth of breast cancer cell expressing the Wilms' Tumour 1 (WT1) gene product, using a WT1 antisense oligonucleotide. WO-A-02/092854 discloses genes that are expressed in breast cancer and are prognostic and therapeutic targets.

As more molecular information is collated, diseases such as breast cancer are being sub-divided according to genetic signatures linked to patient outcome, providing valuable information for the clinician. Emerging novel technologies in molecular medicine have already demonstrated their power in discriminating between disease sub-types that are not recognisable by traditional pathological criteria (Sorlie et al., 2001) and in identifying specific genetic events involved in cancer progression (Srinivas et al., 2002). Further issues need to be addressed in parallel, relating to the efficacy of biomarkers between genders and races, thus large scale screening of a diverse population is a necessity.

The management of breast cancer could be improved by the use of new markers normally expressed only in the breast but found elsewhere in the body, as a result of the disease. Predictors of the activity of the disease would also have valuable utility in the management of the disease, especially those that predict if a ductal carcinoma *in situ* will develop into invasive ductal carcinoma.

### Summary of the Invention

According to a first aspect of the present invention, there is an in vitro method for the detection of the presence of or the risk of breast cancer in a patient, comprising the steps of:
(i) isolating a sample of the patient's genome; and
(ii) detecting the presence or expression of the gene comprised within the sequence identified herein as SEQ ID No. 2, or its complement, or a polynucleotide of at least 15 consecutive nucleotides that hybridises to SEQ ID No.2 (or its complement) under stringent hybridising conditions, wherein the presence or expression of the gene indicates the presence of or the risk of breast cancer.

According to a second aspect of the invention, an isolated polynucleotide comprises the nucleotide sequence identified herein as SEQ ID No. 2, or its complement, or a polynucleotide of at least 15 consecutive nucleotides that hybridises to the sequence (or its complement) under stringent hybridising conditions.

According to a third aspect of the present invention, an isolated peptide comprises the sequence identified herein as SEQ ID No.3, or a fragment thereof of at least 10 consecutive amino acid residues.

According to a fourth aspect of the invention, an antibody has an affinity of at least 10⁻⁶M for a peptide as defined above.

### Description of the Drawings

The invention is described with reference to the accompanying figures, wherein:
Figure 1 shows the results of a screening assay to determine the presence of the gene of interest in different tissues, T represents tumour tissue cDNA and M represents co-excised mammary tissue cDNA from the same donor; and
Figure 2 shows the results of an expression analysis carried out to determine the expression of the gene of interest in different tissue samples.

### Description of the Invention

The present invention is based on the identification of a gene that is expressed in a patient suffering breast cancer. Identification of the gene (or its expressed product) in a sample obtained from a patient indicates the presence of or the risk of breast cancer in the patient.

The invention further relates to reagents such as polypeptide sequences, useful for detecting, diagnosing, monitoring, prognosticating, preventing, imaging, treating or determining a pre-disposition to cancer.

The invention is suitable for the detection of cancer of the breast, in particular an ERα-positive tumour.

The methods to carry out the diagnosis can involve the synthesis of cDNA from mRNA in a test sample, amplifying as appropriate portions of the cDNA corresponding to the gene or a fragment thereof and detecting the product as an indication of the presence of the disease in that tissue, or detecting translation products of the mRNAs comprising gene sequences as an indication of the presence of the disease.

Useful reagents include polypeptides or fragment(s) thereof which may be useful in diagnostic methods such as RT-PCR, PCR or hybridisation assays of mRNA extracted from biopsied tissue, blood or other test samples; or proteins which are the translation products of such mRNAs; or antibodies directed against these proteins. These assays also include methods for detecting the gene products (proteins) in light of possible post-translational modifications that can occur in the body, including interactions with molecules such as co-factors, inhibitors, activators and other proteins in the formation of sub-unit complexes.

The gene associated with breast cancer, is comprised within the polynucleotide sequence shown as SEQ ID No. 1. The putative coding region is shown as SEQ ID No. 2. The expressed product of the gene is identified herein by SEQ ID No. 3. Identification of the gene or its expressed product may be carried out using techniques known for the detection or characterisation of polynucleotides or polypeptides. For example, isolated genetic material from a patient can be probed using short oligonucleotides that hybridise specifically to the target gene. The oligonucleotide probes may be detectably labelled, for example with a fluorophore, so that, upon hybridisation with the target gene, the probes can be detected. Alternatively, the gene, or parts thereof, may be amplified using the polymerase chain reaction, with the products being identified, again using labelled oligonucleotides.

Diagnostic assays incorporating this gene, or associated protein or antibodies include, but are not limited to:
Polymerase chain reaction (PCR)
Reverse transcription PCR (RT-PCR)
Real-time PCR
In-Situ hybridisation
Southern dot blots
Immuno-histochemistry
Ribonuclease protection assay
cDNA array techniques
ELISA

Protein, antigen or antibody arrays on solid supports such as glass or ceramics, useful in binding studies.

Small interfering RNA functional assays.

All of the above techniques are well known to those in the art.

An alternative detection method is to use what are referred to in the art as "Molecular beacons". Molecular beacons are oligonucleotides designed for the detection and quantification of target nucleic acids. The oligonucleotides usually comprise self-hybridising portions that, in the absence of a target nucleic acid, form a stem-loop structure. A fluorescent moiety and a quencher moiety are attached at each end of the oligonucleotide, and are positioned adjacently when the oligonucleotide is in the stem-loop orientation. Fluorescence is effectively prevented by the quencher moiety in this orientation. The loop portion of the oligonucleotide is complementary to a specific target nucleic acid and, in the presence of the target, hybridisation to the target occurs disrupting the stem loop orientation, separating the fluor and quencher, resulting in an increase in detectable fluorescence. The use of the molecular beacons approach to the detection of a gene sequence is disclosed in US 6548254.

The present invention is also concerned with isolated polynucleotides that comprise the sequence identified as SEQ ID No. 2, or its complement, or fragments thereof that comprise at least 15 consecutive nucleotides, preferably 30 nucleotides, more preferably at least 50 nucleotides. Polynucleotides that hybridise to a polynucleotide as defined above, are also within the scope of the invention. Hybridisation will usually be carried out under stringent conditions. Stringent hybridising conditions are known to the skilled person, and are chosen to reduce the possibility of non-complementary hybridisation. Examples of suitable conditions are disclosed in Nucleic Acid Hybridisation, A Practical Approach (B.D. Hames and S.J. Higgins, editors IRL Press, 1985). More specifically, stringent hybridisation conditions include overnight incubation at 42°C in a solution comprising: 50% formamide, 5 x SSC (150 mM NaCL, 15 mM trisodium citrate), 50 mM sodium phosphate (ph7.6), 5 x Denhardt's solution, 10% dextran sulphate and 20 µg/ml denatured, sheared salmon sperm DNA, followed by washing in 0.1 x SSC at about 65°C.

The present invention also relates to isolated polypeptide products of the gene of interest. An isolated polypeptide of the invention comprises the sequence identified herein as SEQ ID No. 3, or a fragment of at least 10 consecutive amino acids thereof, preferably at least 15 consecutive amino acids and more preferably at least 20 amino acids. The polypeptide may be useful in the generation of antibodies or in the development of protein binding molecules that can bind *in vivo* to the protein to inhibit its activity.

The present invention also includes antibodies raised against a polypeptide of the invention. The antibodies will have an affinity for the polypeptide of at least 10⁻⁶M, more preferably, 10⁻⁹M and most preferably at least 10⁻¹¹M. The antibody may be of any suitable type, including monoclonal or polyclonal. Assay kits for determining the presence of the polypeptide antigen in a test sample are also included. In one embodiment, the assay kit comprises a container with an antibody, which specifically binds to the antigen, wherein the antigen comprises at least one epitope encoded by the gene of the invention. These kits can further comprise containers with useful tools for collecting test samples, such as blood, saliva, urine and stool. Such tools include lancets and absorbent paper or cloth for collecting and stabilising blood, swabs for collecting and stabilising saliva, cups for collecting and stabilising urine and stool samples. The antibody can be attached to a solid phase, such as glass or a ceramic surface.

Detection of antibodies that specifically bind to the antigen in a test sample suspected of containing these antibodies may also be carried out. This detection method comprises contacting the test sample with a polypeptide which contains at least one epitope of the gene. Contacting is performed for a time and under conditions sufficient to allow antigen/antibody complexes to form. The method further entails detecting complexes, which contain the polypeptide. The polypeptide complex can be produced recombinantly or synthetically or be purified from natural sources.

In a separate embodiment of the invention, antibodies, or fragments thereof, against the antigen can be used for the detection of image localisation of the antigen in a patient for the purpose of detecting or diagnosing the disease or condition. Such antibodies can be monoclonal or polyclonal, or made by molecular biology techniques and can be labelled with a variety of detectable agents, including, but not limited to radioisotopes.

In a further embodiment, antibodies or fragments thereof, whether monoclonal or polyclonal or made by molecular biology techniques, can be used as therapeutics for the treatment of diseases characterised by the expression of the gene of the invention. The antibody may be used without derivatisation, or it may be derivitised with a cytotoxic agent such as radioisotope, enzyme, toxin, drug, pro-drug or the like.

The term "antibody" refers broadly to any immunologic binding agent such as IgG, IgM, IgA, IgD and IgE. Antibody is also used to refer to any antibody-like molecule that has an antigen-binding region and includes, but is not limited to, antibody fragments such as single domain antibodies (DABS), Fv, scFv etc. The techniques for preparing and using various antibody-based constructs and fragments are well known in the art.

If desired, the cancer screening methods of the present invention may be readily combined with other methods in order to provide an even more reliable indication of diagnosis or prognosis, thus providing a multi-marker test.

The following example illustrates the invention with reference to the accompanying drawings.

### Example

A number of differentially expressed gene fragments were isolated from cDNA populations derived from matched clinical samples of breast cancer patients, using non-isotopic differential display (DDRT-PCR). One of these fragments, referred to herein as DD11, was revealed to be significantly up-regulated in breast tumour tissue samples from a number of donors. The expression profile of this novel molecular marker, its full length and corresponding presumed protein sequence is detailed herein.

### Materials and methods

Differential gene expression between matched pairs of normal mammary and tumour tissue from the same donor was carried out. Tissue samples were obtained, with full ethical approval and informed patient consent, from Pathlore, Peterborough, UK. Following the surgical removal of a tumour, one sample of the tumour tissue was collected, as was a sample from the adjacent, co-excised normal tissue. Messenger RNA was extracted and cDNA subsequently synthesised, using Dynal dT₁₈-tagged Dynabeads and Superscript II reverse transcription protocols, respectively. Differential display reverse transcription PCR (DDRT-PCR) was employed to observe differences between the gene expression profiles of these matched samples, and individual gene transcripts showing up- or down-regulation were isolated and investigated further.

First described by Liang & Pardee (1992), differential display reverse transcription PCR (DDRT-PCR) uses mRNAfrom two or more biological samples as templates for representative cDNA synthesis by reverse transcription, with one of 3 possible anchor primers. Each of the 3 sub-populations was PCR-amplified using its respective anchor primer coupled with one of 80 arbitrary 13-mer primers. This number of primer combinations has been estimated to facilitate the representation of 96% of expressed genes in an mRNA population (Sturtevant, 2000). This population sub-division results in the reduction of the estimated 12,000-15,000 mRNAs expressed in eukaryotic cells to 100-150 transcripts by the end of second strand cDNA synthesis for each primer set. This facilitates the parallel electrophoretic separation and accurate visualization of matched primer sets on a polyacrylamide gel, leading to the identification of gene fragments expressed in one tissue sample but not the other.

Excision and re-amplification of fragments of interest was followed by removal of false positives through reverse Southern dot blotting. This entailed the spotting of each re-amplified fragment onto duplicate nylon membranes (Hybond N+, Amersham Pharmacia Biotech) and hybridising these with either the tumour or normal tissue cDNA population of the donor from which the fragments were derived. Those fragments confirmed as differentially expressed were then direct-sequenced, i.e. without cloning, followed by web-based database interrogation to determine if each gene was novel. Fragments not matching known genes were regarded as potentially representing novel markers for the breast cancer from which they were derived. Further screening of each transcript was performed by either semi-quantitative RT-PCR or real-time PCR, using a suite of matched cDNA populations from a number of breast tumour donors. In all cases, β-actin was used as a constitutive reference gene, for calibrating the cDNA templates and as an internal positive control during PCR. Expression of each putative novel marker gene was performed through the use of gene-specific primer sets on the calibrated templates. Full-length transcripts of the novel gene fragments, including the open reading frame (that piece of the gene that encodes the protein) were then synthesized using a complex process known as 5' RACE (rapid amplification of cDNA ends), which incorporates gene-specific extension and amplification, verifiable by sequencing.

Determination of tissue specificity was assayed using the gene-specific primers from each novel marker against cDNA populations from non-breast tissue, including brain, heart, lymphocytes, spleen, kidney, testis and muscle (obtained from Origene). The DD11 molecular marker was further tested using cDNA populations derived from a more comprehensive panel of 22 human tissue types. These are as follows:

| | |
|---|---|
| Adrenal gland | pooled from 62 donors |
| Bone marrow | pooled from 7 donors |
| Brain, cerebellum | pooled from 24 donors |
| Brain, whole | pooled from one donor |
| Colon* | pooled from one donor |
| Foetal brain | pooled from 59 donors |
| Foetal liver | pooled from 63 donors |
| Heart | pooled from one donor |
| Kidney | pooled from one donor |
| Liver | pooled from one donor |
| Lung | pooled from one donor |
| Placenta | pooled from 7 donors |
| Prostate | pooled from 47 donors |
| Salivary gland | pooled from 24 donors |
| Skeletal muscle | pooled from 2 donors |
| Small intestine* | pooled from one donor |
| Spleen | pooled from 14 donors |
| Testis | pooled from 19 donors |
| Thymus | pooled from 9 donors |
| Thyroid gland | pooled from 65 donors |
| Trachea | pooled from 1 donor |
| Uterus | pooled from 10 donors |

Note that the majority of these samples were part of the Human Total RNA panel II (Clontech), but two samples, marked with asterisks, were obtained as tissue chunks from Pathlore (Peterborough Hospital Tissue Bank) and processed at Randox Laboratories Ltd.

In addition, assays were performed on a range of ethically approved human tumour samples, as obtained through Pathlore. cDNA representative of tumours from ovary, testis, stomach, liver, lung, bladder, colon and pancreas were tested against both β-actin and DD11 by real-time PCR.

In conjunction with novel marker expression analysis, each matched pair of breast tissues was subjected to molecular signature analysis. This entailed using a suite of primers specific to a number of pre-published breast cancer molecular markers in semi-quantitative RT-PCR against each tissue cDNA. The relationship between each molecular marker was determined and tabulated for each sample and used as a reference, against which the novel markers could be compared. This was with the aim of sub-classifying the tumour types to enable the association of novel markers against such sub-types, increasing the power of the diagnostic marker considerably.

### Results and Discussion

Using differential display, a gene fragment, termed DD11, derived from cDNA populations of matched tissue from a breast cancer donor, was observed to have significant up-regulation in the tumour cDNA population in comparison to the corresponding normal tissue cDNA. This product was confirmed as differentially expressed by reverse Southern dot blots. Sequence analysis followed by database interrogation determined that DD11 was not homologous to known genes or proteins in the EMBL and SWISSPROT databases, respectively, so was regarded as potentially novel. It was, however, 100% homologous, after removal of the poly-A tail, to a clone (RP11-875011) from chromosome 8 of the human genome (Accession Number AC107959).

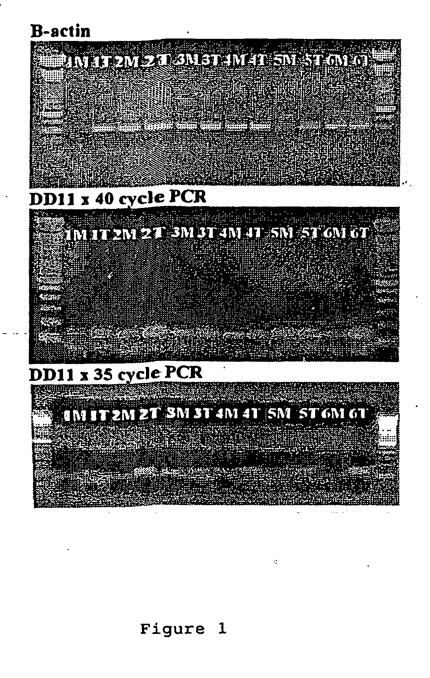

This fragment was further screened using cDNA populations derived from a number of matched breast tumour tissues donated by other patients. Of the donor samples screened, 6 out of 9 exhibited notable increases in expression, confirming DD11 to be a putative molecular marker for the presence of breast tumour (Figure 1). This analysis was substantiated by the molecular signature analysis of all currently available matched breast tissue samples, as follows;

| | | |
|---|---|---|
| Increased in tumour | 10 | 52.6% |
| Increased in normal | 1 | 5.3% |
| No discernable difference | 7 | 36.8% |
| No expression evident | 1 | 5.3% |
| Totals | 19 | 100% |

To facilitate further analysis, 5'-RACE was employed to extend the fragment to include the full open reading frame (ORF) of the gene, plus any 5' non-coding sequence. Using this technique, a presumed full-length product of 513 nucleotides was derived (SEQ ID No. 1), which on subsequent database interrogation, confirmed the previous homology to human chromosome 8, being 100% homologous over the full length of the sequence (513/513). From this sequence, all 6 amino acid reading frames were generated and a putative, small ORF (SEQ ID No. 2) was found in the +2 frame, comprising 47 amino acids, including the stop codon (SEQ ID No. 3). This small protein failed to reveal a high homology to any known proteins in the SWALL database, so is assumed to be novel.

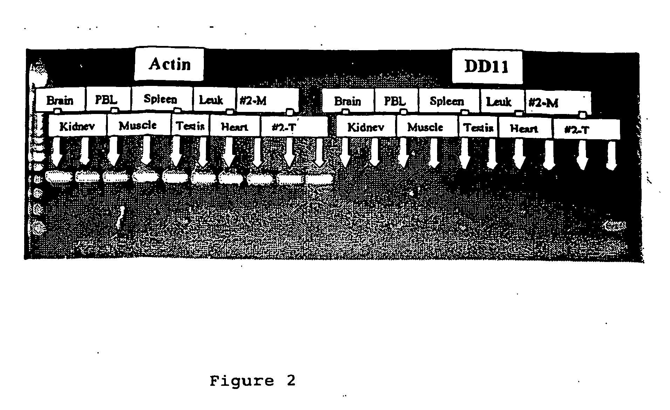

To determine organ specificity, cDNA populations from 8 non-breast human tissues were tested against the DD11 primers, in addition to a matched pair of cDNAs from a breast cancer donor. The same samples were also tested using primers from the constitutive housekeeping gene, β-actin, as a positive control and to calibrate the templates for semi-quantitative PCR analysis. The β-actin product was strongly amplified in all cDNA populations studied, whereas the DD11 product was only detected in the breast tumour sample (Figure 2). This provided further evidence that this novel gene could be a very powerful molecular marker for the presence of a breast tumour.

This molecular markerwas further tested using cDNA populations derived from a panel of 22 human tissue types, both by conventional and real-time PCR analysis. Of those tested, using the Opticon II real-time thermal cycler (MJ Research), DD11 was only detected in samples from placenta and testis. In addition, assays were performed on a range of ethically approved human tumour samples, as obtained through Pathlore, to ascertain whether the marker was breast tumour specific or a less specific tumour marker. cDNA representative of tumours from ovary, testis, colon, stomach, liver, lung, bladder and pancreas were tested against both β-actin and DD11. Of these, DD11 was only detected at a significant level in cDNA derived from testis tumour. The products from these PCR amplifications were verified as DD11 by direct sequencing.

Conventional PCR amplification on a standard thermal cycler, using the combined panels of 22 normal human tissue cDNAs and the 8 tumour cDNA populations, confirmed DD11 to be specific to a very limited number of tissue types. As in the real-time PCR analysis, the testis cDNA population expressed DD11, as did the testis tumour. The only other population showing significant expression of this marker was from the uterus sample. The placenta sample showed a low level of expression, but this was not to the same scale as the testis and uterus samples. Low levels of product were also found in some other tissue samples, but these were considered negligible. This would indicate that of all the samples tested, DD11 is only strongly expressed in those tissues under the influence of reproductive hormones. Specifically, the tissues derived from breast and associated tumour, testis and associated tumour and uterus. Placenta tissue also expresses this marker to a lesser extent.

DD11 also compared very favourably with some of the most highly regarded "standard" breast cancer markers, such as Oestrogen receptor (ERα) and human epidermal growth factor receptor (c-ErbB-2). This is evident both in the molecular signature analysis of all matched breast cancer tissue samples, where expression is similar in both samples from the same patient in many cases and using the target-specific primers against an in-house panel of 30 cDNA populations from human normal and tumour tissue.

### References

DeRisi, J. L., lyer, V. R. and Brown, P. O. 1997. Exploring the metabolic and genetic control of gene expression on a genomic scale. Science. 278: 680-686.
Liang, P. and Pardee, A. B. 1992. Differential display of eukaryotic messenger RNA by means of the polymerase reaction. Science. 257: 967-971.
Pritzker, K. P. 2002 Cancer biomarkers: easier said than done. Clin. Chem. 2002 Aug; 48(8):1147-50.
Salodof MacNeil. 2001. From genes to proteins: The FLEXgene consortium. HMS Beagle. 112: on-line journal.
Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. 2001. Gene expression patterns of breast carcinomas distinguish tumor subclasses with clinical implications. Proc Natl Acad Sci U S A. Sep 11;98(19):10869-74.
Srinivas PR, Verma M, Zhao Y, Srivastava S. 2002. Proteomics for cancer biomarker discovery. Clin Chem. Aug;48(8):1160-9.
Sturtevant, J. Applications of differential-display reverse transcription-PCR to molecular pathogenesis and medical mycology. Clin Microbiol Rev. 2000 Jul;13(3):408-27.
Yousef et al., 2002 Yousef GM, Scorilas A, Kyriakopoulou LG, Rendl L, Diamandis M, Ponzone R, Biglia N, Giai M, Roagna R, Sismondi P, Diamandis EP. Human kallikrein gene 5 (KLK5) expression by quantitative PCR: an independent indicator of poor prognosis in breast cancer. Clin Chem. 2002 Aug;48(8):1241-50.
Zong, Q., Schummer, M., Hood, L. and Morris, D. R. 1999. Messenger RNA translation state: the second dimension of high-throughput expression screening. Proc. Natl. Acad. Sci. 96: 10632-10636.

## Claims

1. An *in vitro* method for the detection of the presence of or the risk of breast cancer in a patient comprising the steps of:
(i) isolating a sample of the patient's genome; and
(ii) detecting the presence or expression of the gene comprised within the sequence identified herein as SEQ ID No. 2, or its complement, or a polynucleotide of at least 15 consecutive nucleotides that hybridises to SEQ ID No. 2 (or its complement) under stringent hybridising conditions,
wherein the presence or expression of the gene indicates the presence of or the risk of breast cancer.

2. A method according to claim 1, wherein the genome sample is obtained from breast tissue.

3. A method according to claim 1 or claim 2, wherein detection is carried out by amplifying the gene using the polymerase enzyme.

4. An isolated polynucleotide comprising the nucleotide sequence identified herein as SEQ ID No. 2, or its complement, or a polynucleotide of at least 15 consecutive nucleotides that hybridises to the sequence (or its complement) under stringent hybridising conditions.

5. Use of a polynucleotide according to claim 4, in an in vitro diagnostic assay to test for the risk of breast cancer in a patient.

6. A peptide comprising the sequence identified herein as SEQ ID No. 3, or a fragment thereof of at least 10 consecutive amino acid residues.

7. An antibody having an affinity of at least 10⁻⁶M for the peptide of claim 6.

## Patentansprüche

1. In-vitro-Verfahren zum Nachweis des Vorliegens oder des Risikos von Brustkrebs in einem Patienten, umfassend die Schritte des:
(i) Isolierens einer Probe des Patientengenoms und
(ii) Nachweisens des Vorliegens oder der Expression des Gens, das in der hier als SEQ ID No. 2 bezeichneten Sequenz enthalten ist, oder ihres Gegenstrangs, oder eines Polynukleotids von mindestens 15 aufeinanderfolgenden Nukleotiden, das unter stringenten Hybridisierungsbedingungen an SEQ ID No. 2 (oder ihren Gegenstrang) hybridisiert,
wobei das Vorliegen oder die Expression des Gens auf das Vorliegen oder das Risiko von Brustkrebs hindeutet.

2. Verfahren gemäß Anspruch 1, wobei die Genomprobe aus Brustgewebe gewonnen wird.

3. Verfahren gemäß Anspruch 1 oder Anspruch 2, wobei der Nachweis geführt wird, indem das Gen unter Verwendung des Enzyms Polymerase amplifiziert wird.

4. Isoliertes Polynukleotid, welches die hier als SEQ ID No. 2 bezeichnete Nukleotidsequenz oder ihren Gegenstrang oder ein Polynukleotid von mindestens 15 aufeinanderfolgenden Nukleotiden, das unter stringenten Hybridisierungsbedingungen an die Sequenz (oder ihren Gegenstrang) hybridisiert, umfasst.

5. Verwendung eines Polynukleotids gemäß Anspruch 4 in einem In-vitro-Diagnostiktest zum Untersuchen auf das Brustkrebsrisiko in einem Patienten.

6. Peptid, weiches die hier als SEQ ID No. 3 bezeichnete Sequenz oder ein Fragment davon von mindestens 10 aufeinanderfolgenden Aminosäurenresten umfasst.

7. Antikörper mit einer Affinität von mindestens 10⁻⁶ M für das Peptid nach Anspruch 6.

## Revendications

1. Procédé *in vitro* de détection de la présence ou du risque de cancer du sein chez un patient, comprenant les étapes de :
(i) isolement d'un échantillon du génome du patient ; et
(ii) détection de la présence ou de l'expression du gène compris à l'intérieur de la séquence identifiée ici comme étant SEQ ID No. 2, ou de son complément, ou d'un polynucléotide d'au moins 15 nucléotides consécutifs qui s'hybride à SEQ ID No. 2 (ou à son complément) dans des conditions d'hybridation stringentes,
où la présence ou l'expression du gène indique la présence ou le risque de cancer du sein.

2. Procédé selon la revendication 1, où l'échantillon génomique est obtenu à partir de tissu de sein.

3. Procédé selon la revendication 1 ou la revendication 2, où la détection est effectuée par amplification du gène au moyen de l'enzyme polymérase.

4. Polynucléotide isolé comprenant la séquence nucléotidique identifiée ici comme étant SEQ ID No. 2, ou son complément, ou un polynucléotide d'au moins 15 nucléotides consécutifs qui s'hybride à la séquence (ou à son complément) dans des conditions d'hybridation stringentes.

5. Utilisation d'un polynucléotide selon la revendication 4, dans un dosage diagnostic *in vitro* pour évaluer le risque de cancer du sein chez un patient.

6. Peptide comprenant la séquence identifiée ici comme étant SEQ ID No. 3, ou un fragment de celle-ci d'au moins 10 résidus acides aminés consécutifs.

7. Anticorps ayant une affinité d'au moins 10⁻⁶ M pour le peptide selon la revendication 6.


### SEQUENCE LISTING

<110> Randox Laboratories Ltd.
<120> Diagnosis of Risk of Breast Cancer
<130> JWJ01047WO
<150> 0320648.9
   <151> 2003-09-03
<160> 3
<170> PatentIn version 3.1
<210> 1
   <211> 513
   <212> DNA
   <213> Homo sapiens
<400> 1
   tttatggtca taagcttaga aaatcctttg cccaacataa aataagagaa ctctaatttc      60
   ttagggagat ttttattaaa tgattagatt tgtagcatat agttgtataa aataagatga     120
   actctaattt cttagggagg ttttattaaa tgattagatt tgtagcatat catcgtgtaa     180
   agtacatgga cattattttt gatatagaaa gtgtagtgtt ccccttcatt gttctgagtt     240
   actctcatct gtccaacccc agcgagccac tgattattcc ctttctctga actttgtggt     300
   gtttatggaa gcttcattcc gtagcacgaa ggcgtcaatc attaatctcg ggtgattagt     360
   cctcaggcat ctccctgctc tgagctgagg ggtgtggtag tgttgaaagt tgcagtgctc     420
   tgatcacgtg gttggttcga ctggtaactg gtccctctct ggcaagagcc acctcatcag     480
   tatcaactca ggaatgctgg aaatcatttt atg                                  513
<210> 2
   <211> 138
   <212> DNA
   <213> Homo sapiens
<220>
   <221> CDS
   <222> (1)..(138)
   <223>
<400> 2
   atg att aga ttt gta gca tat cat cgt gta aag tac atg gac att att      48
   ttt gat ata gaa agt gta gtg ttc ccc ttc att gtt ctg agt tac tct      96
   cat ctg tcc aac ccc agc gag cca ctg att att ccc ttt ctc          138
<210> 3
   <211> 46
   <212> PRT
   <213> Homo sapiens
<400> 3